# FASCICULE DE BREVET EUROPEEN

(11) **EP 0 645 134 B1**
(45) Date de publication et mention de la délivrance du brevet: **07.10.1998**
(21) Numéro de dépôt: 94401743.3
(22) Date de dépôt: 28.07.1994
(51) Int. Cl.: A61K 7/043, A61K 7/48

(54) **Composition cosmétique comprenant une huile époxydée comme plastifiant**
Kosmetisches Mittel enthaltend ein epoxydiertes Öl als Weichmacher
Cosmetic composition containing as plastifiant an epoxylated oil

(30) Priorité: 28.09.1993 FR 9311501
(43) Date de publication de la demande: 29.03.1995
(73) Titulaire: L'OREAL, 75008 Paris (FR)
(72) Inventeur: Mellul, Myriam, F-94240 L'Hay Les Roses (FR); De La Poterie, Valérie, F-77820 Le Hatelet en Brie (FR)
(74) Mandataire: Dodin, Catherine

(56) Documents cités:
- FR-A- 2 591 104
- S.T.N., Serveur de Bases de Données, KARLSRUHE, DE, Fichier Chemical Abstracts, vol 120, n 194059, * résumé *

## Description

La présente invention a pour objet une composition cosmétique filmogène, par exemple de vernis à ongles, incolore ou colorée, contenant outre les constituants habituels, une huile époxydée comme plastifiant.

Parmi les principales caractéristiques que doivent posséder les compositions cosmétiques filmogènes telles que les vernis à ongles, on doit tout particulièrement citer l'absence d'irritation du support, peau, ongles et/ou cheveux, une bonne application, l'obtention d'un film homogène d'excellente brillance, un temps de séchage du film rapide, mais aussi une bonne adhérence sur la surface du support, une certaine flexibilité et une bonne résistance du film, par exemple en vue d'éviter les craquelures et son écaillement.
Ces caractéristiques sont généralement dénommées : caractéristiques cosmétiques.
De façon générale, on utilise à l'heure actuelle, par exemple dans les vernis à ongles, pour conférer une bonne adhérence et une bonne flexibilité du film, des matières filmogènes telles que la nitrocellulose associée, si nécessaire, à un autre polymère tel qu'une résine toluène sulfonamide formaldéhyde ou une résine alkyde et des plastifiants (voir à ce sujet notamment le document FR-A-2679445). Les plastifiants couramment utilisés sont les plastifiants de type phtalate comme le phtalate de dibutyle, de type citrate comme l'acétyl citrate de tributyle, de type ester de glycol comme l'ester de néopentylglycol ou de propylène glycol, de type benzoate de glycéryle et enfin le camphre.
Ces agents plastifiants permettent de régler la flexibilité du film sans affaiblir sa résistance physique.
De nos jours, il est préférable d'utiliser dans les vernis d'autres plastifiants que les phtalates pour des raisons d'allergie mais aussi que le camphre qui par sa volatilité ne permet pas d'avoir des caractéristiques de vernis constantes. Par ailleurs, il est préférable, et pour encore des raisons d'allergie, d'éviter l'utilisation d'un polymère source de formaldéhyde.
En vue de l'application du vernis sur les ongles, on utilise à l'heure actuelle des compositions liquides contenant une grande quantité de solvant organique (en général environ 70 % en poids de la composition totale). Or, pour des raisons de pollution de l'environnement, on cherche à diminuer cette quantité de solvants.
Par ailleurs, la formulation des vernis à ongles est délicate car la composition est un ajustement judicieux entre les différents constituants. Ainsi, le changement d'un de ces constituants peut modifier considérablement les propriétés cosmétiques du vernis comme l'application, la brillance, le séchage, la dureté ou la tenue du film, rendant ce vernis impropre à la consommation.

Pour ces raisons, les formulations de vernis à ongles actuellement disponibles sur le marché sont quasi-identiques, voire identiques.

L'invention a justement pour objet une nouvelle composition cosmétique permettant notamment de remédier aux inconvénients ci-dessus, tout en présentant d'excellentes propriétés cosmétiques.
Elle permet en particulier la réduction, voire l'élimination des plastifiants usuels, ainsi que la réduction de la quantité de solvant.
Elle permet en outre l'obtention de nouvelles compositions hypoallergéniques.

De façon surprenante, il a été trouvé qu'on pouvait utiliser une huile époxydée comme plastifiant dans les compositions cosmétique à milieu organique, sans modifier les propriétés cosmétiques du film.
Cette huile peut être utilisée seule ou éventuellement associée à un autre plastifiant connu de l'homme de l'art.

De façon plus précise, l'invention se rapporte à une composition cosmétique selon la revendication 1.

Cette huile peut être synthétique ou avantageusement naturelle.
Comme huile naturelle, on peut citer les huiles végétales comme l'huile de soja époxydée, l'huile de lin époxydée et de préférence l'huile de Vernonia époxydée ainsi que leurs mélanges.
L'huile de Vernonia, et plus exactement de *Vernonia galamensis*, est du type triglycéride dont la composition générale en acide gras est la suivante :

| | | |
|---|---|---|
| - acide vernolique | C 18:1 (insaturée) | 72,0 - 80,0 % |
| - acide palmitique | C 16:0 | 2,0 - 3,0 % |
| - acide stéarique | C 18:0 | 2,0 - 4,0 % |
| -acide oléique | C 18:1 | 4,0 - 6,0 % |
| - acide linoléique | C 18:2 | 11,0 - 14,0 % |

L'huile de Vernonia utilisée dans la présente invention est une huile végétale raffinée, obtenue par pression à froid de graines de Vernonia pillées désactivées. Cette huile raffinée est un liquide homogène transparent de couleur jaune parfaitement soluble dans la plupart des solvants organiques.

L'intérêt de cette huile est sa faible viscosité (de l'ordre de 300 mPa.s ) qui permet de l'introduire en plus ou moins grande quantité sans modifier considérablement, par rapport aux compositions classiques, la viscosité du milieu. Cette faible viscosité permet en outre de diminuer, par rapport aux compositions connues, la quantité de solvant.
A ce jour, la seule utilisation connue de l'huile de Vernonia est, en tant que diluant, dans des peintures (voir à cet effet l'article de S. DIRLIKOV et al. "Increasing high-solids with a weed", Agro-industrial Finishing, p. 17-19).

La composition de l'invention contient en particulier, en poids, de 0,5 % à 25 % de plastifiant, de 5 % à 35 % de matière filmogène autre que le plastifiant et 40 % à 90 % de milieu solvant et de préférence de 3 à 15 % de plastifiant, de 15 % à 35 % de matière filmogène autre que le plastifiant et de 50 % à 82 % de milieu solvant.
Avec ces compositions, il est possible d'obtenir des extraits secs allant de 20 % à 50 % en poids et de préférence entre 25 % et 40 %.

En outre, l'huile époxydée peut être présente à des pourcentages supérieurs à 9 % en poids de la composition totale dans des vernis à haut extrait sec, sans modifier considérablement la viscosité, donnant ainsi des vernis à teneur en produits volatils réduits et présentant des caractéristiques cosmétiques excellentes.

Les plastifiants utilisables en association avec l'huile époxydée sont par exemple ceux cités dans le document FR-A-2679445 tels que les phtalates de dibutyle, de dioctyle, de di-isobutyle, de diméthoxyéthyle les benzoates de benzyle, de glycéryle les citrates de triéthyle, de tributyle, I'acétyl-citrate de tributyle les phosphates de tributyle, de triphényle les glycols le camphre ainsi que leurs dérivés et leurs mélanges. Ces plastifiants représentent au plus 50 % en poids de la quantité totale de plastifiant.

Les matières filmogènes de l'invention comprennent en particulier la nitrocellulose éventuellement associée à une résine alkyde, polyuréthanne, acrylique, vinylique, aryl-sulfonamide formaldéhyde ou aryl-sulfonamide époxy et de façon générale toute résine compatible avec le milieu.
Ces résines permettent d'augmenter le pouvoir filmogène de la nitrocellulose et améliorent le brillant ainsi que l'adhérence des films.

La résine associée à la nitrocellulose est notamment la résine toluène-sulfonamide-urée-formaldéhyde, plus connue sous les dénominations commerciales de "Santolite MHP", "Santolite MS 80 %" et "Ketjenflex MS80", pour son excellente propriété filmogène ou de préférence les résines alkydes par exemple du type alkydeglycérophtalique (BECKOSOL ODE). Les résines alkydes ont l'avantage de ne pas contenir de formaldéhyde et d'être hypoallergéniques.

Aussi de façon avantageuse, la composition de l'invention contient principalement de la nitrocellulose, une résine alkyde, de l'huile de Vernonia et un milieu solvant. Cette composition présente d'excellentes caractéristiques cosmétiques.

Il est en outre possible de remplacer la nitrocellulose par une résine polyvinylique telle que le butyrale de polyvinyle.

Selon l'invention, le milieu solvant est essentiellement constitué par un mélange de divers solvants organiques volatils, ceci en vue d'obtenir des temps de séchage relativement courts.
Les solvants utilisés doivent être compatibles avec les résines utilisées afin d'assurer leur dissolution. Ces solvants sont ceux classiquement utilisés dans les compositions cosmétiques. Parmi ces solvants, on peut citer les cétones, comme l'acétone, la méthyl-éthylcétone, la méthyl-isobutyl-cétone les éthers de glycol ; les alcools comme l'éthanol, le n-butanol, le n-propanol, l'isopropanol ; les acétates comme l'acétate de butyle, d'éthyle, d'isopropyle, I'acétate de méthoxy-2-éthyle les hydrocarbures linéaires ou ramifiés tels que l'hexane ou l'octane ; ou encore les hydrocarbures aromatiques tels que le xylène et le toluène.
De préférence, on utilise les acétates de butyle et d'éthyle.

Lorsque la composition cosmétique se présente sous forme d'un vernis à ongles coloré, on utilise alors au moins un pigment de nature organique ou inorganique.
Parmi les pigments organiques, on peut citer les D & C Red n° 10, 11, 12 et 13, le D & C Red n° 7, les D & C Red n° 5 et 6, les D & C Red n° 34, des laques telles que la laque D & C Yellow n° 5 et la laque D & C Red n° 2. On peut aussi citer comme pigment organique la guanine.
Parmi les pigments inorganiques, on peut citer le dioxyde de titane, l'oxychlorure de bismuth, l'oxyde de fer brun et les oxydes de fer rouge.
Selon cette forme de réalisation, les pigments sont généralement présents dans une proportion allant de 0,01 % à 2,5 % en poids par rapport au poids total du vernis.

Par ailleurs, en vue d'éviter la sédimentation des pigments, certains agents rhéologiques thixotropes peuvent être employés, tels que les argiles du type "Bentone 27", "Bentone 38" ou montmorillonite. Ces agents servent en outre d'épaississants.
Les vernis à ongles selon l'invention peuvent par ailleurs contenir des adjuvants couramment utilisés dans les vernis à ongles. Parmi ces adjuvants, on peut citer les filtres U.V. tels que les dérivés de benzophénone et le 2-cyano-3, 3-diphényl acrylate d'éthyle, des colorants mais aussi des silicones et des agents fluorés. Ces adjuvants sont utilisés à une concentration au plus égale à 1 % en poids par rapport au poids total du vernis.

Les compositions selon l'invention peuvent être principalement utilisées en tant que vernis à ongles.
On peut également envisager de les utiliser en tant que produit de maquillage, mascara par exemple, ou produit capillaire. Dans ce cas, les compositions peuvent contenir en outre les constituants usuellement utilisés dans ces applications, constituants bine connus de l'homme du métier.

On va maintenant donner à titre d'illustration et sans aucun caractère limitatif plusieurs exemples de composition de vernis à ongles selon l'invention ainsi que des exemples comparatifs conformes à l'art antérieur.
Toutes les compositions ci-après ont été obtenues à température ambiante, en solubilisant la nitrocellulose dans le milieu solvant (acétate), en ajoutant l'huile époxydée puis la résine alkyde ou formaldéhyde, puis en dispersant les additifs solides tels que les agents rhéologiques du type argile ainsi que l'acide citrique et les pigments.
La nitrocellulose est utilisée en dissolution dans de l'isopropanol à une concentration de 70 % en poids. La résine alkyde (BECKOSOL ODE) et la résine formaldéhyde (Santolite Ketjenlex MS80) sont dissoutes respectivement à 70 % en poids dans le xylène et 80 % en poids dans l'acétate de butyle.
Les compositions sont présentées sous forme de tableaux I et II et à chaque composition on indique la quantité d'extrait sec correspondant, leur temps de séchage et leur viscosité ainsi que la dureté, la brillance et l'énergie de surface des films obtenus. Les tableaux I et II ont trait respectivement à des vernis incolores et des vernis colorés en rouge par du monochrome DC Red n°7. Ce monochrome a été utilisé en dispersion de 4 % en poids dans la base incolore témoin (échantillon A).

La dureté est déterminée en surface en mesurant le temps de balancement d'un pendule au contact du film elle doit être comprise entre 20 s et 150 s et de préférence entre 30 s et 80 s pour être satisfaisante. Elle est mesurée dans une enceinte thermostatée à 30° C avec un degré constant d'humidité 70 % (RH).
La brillance correspond au coefficient de réflexion lumineux du film elle doit être supérieure à 75 % et de préférence être aux environs de 90 %.
L'énergie de surface caractérise la surface du film et notamment sa capacité au mouillage du support.
Le temps de séchage est mesuré en atmosphère contrôlée en température (30 °C) et en humidité (50 % RH) à l'aide d'un appareil à bille à déplacement circulaire (Braive Instruments), passant sur le film de vernis étalé sur une plaque de verre, d'une épaisseur de 300 µm. Notons que le temps mesuré selon cette technique est reproductible mais supérieur au temps de séchage sur ongle.

Dans le tableau I, on constate que les caractéristiques des films obtenus à partir des compositions B, D et G de l'invention sont comparables à celles obtenues à partir de la composition témoin A, conforme à l'art antérieur et qui contient du Citroflex comme plastifiant.
La composition C conduit à un extrait sec trop dur car la quantité d'huile de Vernonia est insuffisante.
La composition E montre une baisse de la brillance et une augmentation de la dureté par rapport aux compositions B, C et D, ce qui montre que l'huile de Vernonia est meilleure que l'huile de lin. Notons cependant que l'huile de lin utilisée ici était non-époxydée.
La composition H, pour un extrait sec plus élevé que celui des compositions A à G, indique une insuffisance d'huile de Vernonia du fait de la viscosité et de la dureté élevée. En augmentant la quantité d'huile jusqu'à plus de 9 % en poids de la composition, on obtient, à extrait sec comparable, une composition I de l'invention pour laquelle la viscosité décroît et la dureté du film diminue. En comparant la composition I à la composition témoin J de l'art antérieur, on constate que leur viscosité et leur énergie de surface sont comparables. En revanche, la composition I de l'invention est plus brillante que celle de l'art antérieur.
Ainsi, l'huile de Vernonia est un bon plastifiant des vernis à haut extrait sec.

Dans le tableau II, les échantillons Ac, Bc, Cc et Dc correspondent aux échantillons A, B, C et D auxquels on a ajouté le pigment DC Red n°7. D'après ce tableau II on constate que l'ajout d'un pigment aux compositions B, C, D ne modifie pas ou peu les propriétés du vernis à ongles. La brillance, bien que légèrement plus faible que celle des compositions incolores, en raison du pigment, est tout à fait acceptable. En outre, ces compositions colorées ne présentent, après 6 mois de fabrication, aucune sédimentation du pigment et une bonne stabilité.

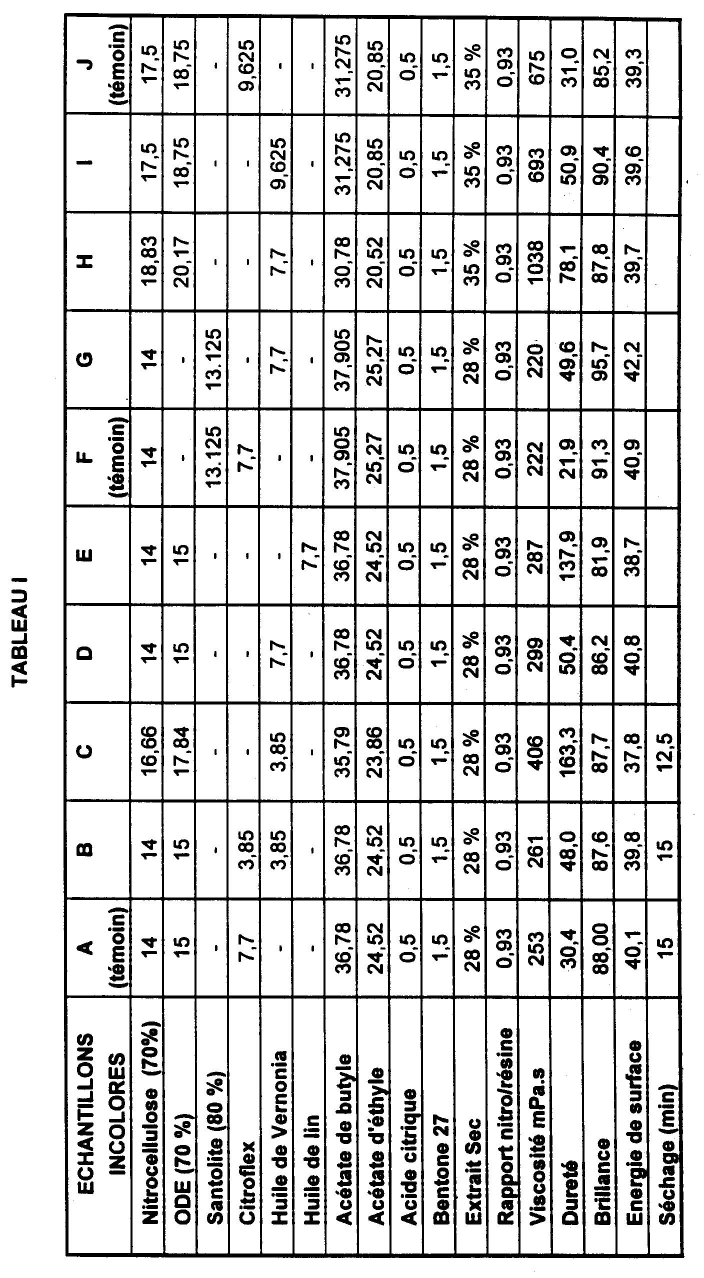

**TABLEAU II**

| **ECHANTILLONS COLORES** | **Ac (témoin)** | **Bc** | **Cc** | **Dc** |
|---|---|---|---|---|
| **Nitrocellulose (70%)** | 12,6 | 12,6 | 14,994 | 12,6 |
| **ODE (70%)** | 13,5 | 13,5 | 16,056 | 13,5 |
| **Citroflex** | 6,93 | 3,465 | - | - |
| **Huile de Vernonia** | - | 3,465 | 3,465 | 6,93 |
| **Acétate de butyle** | 33,102 | 33,102 | 32,211 | 33,102 |
| **Acétate d'éthyle** | 22,068 | 22,068 | 21,474 | 22,068 |
| **Acide citrique** | 0,45 | 0,45 | 0,45 | 0,45 |
| **Bentone 27** | 1,35 | 1,35 | 1,35 | 1,35 |
| **Monochrome DC Red n° 7** | 10 | 10 | 10 | 10 |
| **Extrait Sec** | 28 % | 28 % | 28 % | 28 % |
| **Rapport nitro/résine** | 0,93 | 0,93 | 0,93 | 0,93 |
| **Dureté** | 32,8 | 45,7 | 156,6 | 62,0 |
| **Brillance** | 84,7 | 84,8 | 84,7 | 84,0 |
| **Energie de surface** | 40,6 | 39,4 | 39,1 | 39,9 |

## Revendications

1. Composition de vernis à ongles contenant au moins une matière filmogène, au moins un plastifiant et un milieu solvant, caractérisée en ce que le plastifiant comprend au moins une huile époxydée, le plastifiant étant présent en une teneur en poids allant de 0,5 % à 25 % en poids, ladite composition étant apte à former un film ayant une dureté comprise entre 20 s et 150 s.

2. Composition selon la revendication 1, caractérisée en ce que l'huile est une huile naturelle.

3. Composition selon la revendication 1 ou 2, caractérisée en ce que l'huile est de l'huile de Vernonia.

4. Composition selon l'une quelconque des revendications précédentes, caractérisée en ce qu'elle contient, en poids, de 0,5 % à 25 % de plastifiant, de 5 % à 35 % de matière filmogène autre que le plastifiant et 40 % à 90 % de milieu solvant.

5. Composition selon l'une quelconque des revendications précédentes, caractérisée en ce qu'elle contient, en poids, de 3 % à 15 % de plastifiant, de 15 % à 35 % de matière filmogène autre que le plastifiant et de 50 % à 82 % de milieu solvant.

6. Composition selon l'une quelconque des revendications précédentes, caractérisée en ce qu'elle contient de la nitrocellulose comme matière filmogène.

7. Composition selon l'une quelconque des revendications précédentes, caractérisée en ce que la matière filmogène comprend en outre une résine choisie parmi les résines aryl-sulfonamide formaldéhyde et les résines alkyde.

8. Composition selon la revendication 1, caracterisée en ce qu'elle contient principalement de la nitrocellulose, une résine alkyde, de l'huile de Vernonia et un milieu solvant.

9. Composition selon l'une quelconque des revendications précédentes, caractérisée en ce qu'elle contient au moins un pigment.

10. Composition selon la revendication 9, caractérisée en ce que le pigment est présent à une concentration de 0,01 % à 2,5 % en poids par rapport au poids total de la composition.

## Claims

1. Nail varnish composition containing at least one film-forming material, at least one plasticizer and a solvent medium, characterized in that the plasticizer comprises at least one epoxidized oil, the plasticizer being present in a weight content ranging from 0.5% to 25% by weight, the said composition being capable of forming a film with a hardness of between 20 s and 150 s.

2. Composition according to Claim 1, characterized in that the oil is a natural oil.

3. Composition according to Claim 1 or 2, characterized in that the oil is Vernonia oil.

4. Composition according to any one of the preceding claims, characterized in that it contains, by weight, from 0.5% to 25% of plasticizer, from 5% to 35% of film-forming material other than the plasticizer and 40% to 90% of solvent medium.

5. Composition according to any one of the preceding claims, characterized in that it contains, by weight, from 3% to 15% of plasticizer, from 15% to 35% of film-forming material other than the plasticizer and from 50% to 82% of solvent medium.

6. Composition according to any one of the preceding claims, characterized in that it contains nitrocellulose as film-forming material.

7. Composition according to any one of the preceding claims, characterized in that the film-forming material comprises, in addition, a resin chosen from arylsulphonamide-formaldehyde resins and alkyd resins.

8. Composition according to Claim 1, characterized in that it contains mainly nitrocellulose, an alkyd resin, Vernonia oil and a solvent medium.

9. Composition according to any one of the preceding claims, characterized in that it contains at least one pigment.

10. Composition according to Claim 9, characterized in that the pigment is present at a concentration of 0.01% to 2.5% by weight relative to the total weight of the composition.

## Patentansprüche

1. Nagellackzusammensetzung, die mindestens einen Filmbildner, mindestens einen Weichmacher und ein Lösungsmittelmedium enthält,
dadurch **gekennzeichnet,** daß
der Weichmacher mindestens ein epoxidiertes Öl umfaßt, wobei der Weichmacher in einem Gewichtsanteil von 0,5 bis 25 Gew.-% vorliegt und wobei die Zusammensetzung befähigt ist, einen Film zu bilden, der in 20 bis 150 s hart ist.

2. Zusammensetzung nach Anspruch 1, dadurch **gekennzeichnet,** daß das Öl ein natürliches Öl ist.

3. Zusammensetzung nach Anspruch 1 oder 2, dadurch **gekennzeichnet,** daß das Öl das Vernoniaöl ist.

4. Zusammensetzung nach einem der vorhergehenden Ansprüche, dadurch **gekennzeichnet,** daß sie 0,5 bis 25 Gew.-% Weichmacher, 5 bis 35 Gew.-% filmbildendes Material neben dem Weichmacher und 40 bis 90 Gew.-% Lösungsmittelmedium enthält.

5. Zusammensetzung nach einem der vorhergehenden Ansprüche, dadurch **gekennzeichnet,** daß sie 3 bis 15 Gew.-% Weichmacher, 15 bis 35 Gew.-% filmbildendes Material neben dem Weichmacher und 50 bis 82 Gew.-% Lösungsmittelmedium enthält.

6. Zusammensetzung nach einem der vorhergehenden Ansprüche, dadurch **gekennzeichnet,** daß sie als filmbildendes Material Nitrocellulose enthält.

7. Zusammensetzung nach einem der vorhergehenden Ansprüche, dadurch **gekennzeichnet,** daß der Filmbildner ferner ein Harz umfaßt, das unter den Arylsulfonamid-Formaldehyd-Harzen und den Alkydharzen ausgewählt ist.

8. Zusammensetzung nach Anspruch 1, dadurch **gekennzeichnet,** daß sie hauptsächlich Nitrocellulose, ein Alkydharz, Vernoniaöl und ein Lösungsmittelmedium enthält.

9. Zusammensetzung nach einem der vorhergehenden Ansprüche, dadurch **gekennzeichnet,** daß sie mindestens ein Pigment enthält.

10. Zusammensetzung nach Anspruch 9, dadurch **gekennzeichnet,** daß das Pigment in einer Konzentration von 0,01 bis 2,5 Gew.-%, bezogen auf das Gesamtgewicht der Zusammensetzung, vorliegt.
